# EUROPÄISCHE PATENTSCHRIFT

(11) **EP 0 559 870 B1**
(45) Veröffentlichungstag und Bekanntmachung des Hinweises auf die Patenterteilung: **28.08.1996**
(21) Anmeldenummer: 92920460.0
(22) Anmeldetag: 25.09.1992
(51) Int. Cl.: A61B 17/68

(54) **PLATTE ZUR OSTEOSYNTHESE**
OSTEOSYNTHESIS PLATE
PLAQUE POUR L'OSTEOSYNTHESE

(30) Priorität: 26.09.1991 DE 4132021
(43) Veröffentlichungstag der Anmeldung: 15.09.1993
(73) Patentinhaber: Clasbrummel, Bernhard, D-33415 Verl (DE)
(72) Erfinder: Clasbrummel, Bernhard, D-33415 Verl (DE)
(74) Vertreter: Haft, von Puttkamer, Berngruber, Czybulka
(86) Internationale Anmeldenummer: DE9200826
(87) Internationale Veröffentlichungsnummer: WO9306789

(56) Entgegenhaltungen:
- EP-A- 0 450 423
- WO-A-88/02618
- CH-A- 335 797
- FR-A- 1 239 266

## Beschreibung

Die vorliegende Erfindung betrifft eine Platte zur Osteosynthese frakturierter Röhrenknochen, die mit einer Vielzahl von Schrauben versehen ist zur Verankerung in den miteinander zu verbindenden Fragmenten, entsprechend der CH-A-0 335 797.

Schwerverletzte Personen mit Knochenbrüchen werden in der Unfallchirurgie häufig osteosynthetisch behandelt. Als Osteosyntheseform kann eine Platte mit Schrauben, ein Marknagel oder ein Fixateur Externe verwendet werden.

Ein Fixateur Externe wird dazu verwendet, um nach einer Fraktur oder Osteotomie eines Knochens, insbesondere des in einem Bein oder Arm eines Patienten vorhandenen Knochens, die Knochenfragmente mit Stiften zu fixieren, die von der Außenseite des Körpers her durch die Haut hindurch in die Knochenstücke eingesetzt und dort verschraubt werden. Die Stifte sind mit außerhalb des Körpers gelegenen Haltern verbunden, die wiederum in einem Stellelement aufgenommen sind. Am Fixateur kann ein Antrieb vorgesehen sein, der dafür sorgt, daß sich die beidseits der Frakturstelle angeordneten Stifte relativ zueinander hin- und herbewegen, wodurch auch die Knochenfragmente, in denen die Stifte eingebracht sind, hin- und herbewegt werden. Die Amplituden liegen dabei im Millimeterbereich.

Es konnte von dem Erfinder festgestellt werden, daß durch eine derartige kontrollierte mechanische Stimulation eine Verkürzung der Knochenheilungszeit erreicht werden kann, die besonders ausgeprägt ist, wenn die Stimulationsamplitude und die Stimulationskraft während des Heilungsvorganges in Abhängigkeit von der Festigkeitsentwicklung der zusammenwachsenden Knochen geregelt wird.

Zu Beginn eines Heilungsvorganges einer Knochenfraktur bildet sich nämlich an den gegenüberliegenden Knochenbruchstellen zunächst relativ weiches, nach und nach verknorpelndes und später verknöcherndes Regenerationsgewebe, dessen Wachstum durch die Einleitung von Mikrobewegungen, wie insbesondere Untersuchungen an Tibiafrakturen zeigten, beschleunigt werben kann. So wurde festgestellt, daß derartig behandelte Patienten ihre gebrochenen Unterschenkel im Mittel sechs Wochen früher belasten können als dies bei Patienten der Fall war ohne die Einleitung von Mikrobewegungen über einen Fixateur Externe in den Läsionsbereich.

Anstelle eines Fixateur Externe kann auch zur Frakturheilung die sogenannte biologische Plattenosteosynthese verwendet werden, die sich insbesondere zur Heilung von Mehrfragmentfrakturen durchgesetzt hat; die Bezeichnung biologische Plattenosteosynthese wird dabei üblicherweise im Zusammenhang mit Verplattungen langstreckiger oder mehrsegmentaler Schaftfrakturen großer Röhrenknochen verwendet. Dabei sollen die Abläufe der Frakturheilung so wenig wie möglich irritiert werden, d.h. daß die Platte nach Korrektur von Achsabweichung und Drehfehlern die Hauptfragmente längengerecht miteinander verbinden muß, wobei die Frakturzone nur so weit freigelegt werden darf, daß die Platte ohne weitere Schädigung der Weichteile und der Vascularität der Frakturfragmente angebracht werden kann.

Aus der genannten CH-A-0 335 797 ist es bekannt, die Osteosyntheseplatte in zwei in Langsrichtung hintereinander angeordnete Plattenteile aufzuteilen, die über einen Teleskopmechanismus miteinander verbunden sind, welche axiale Relativbewegungen der beiden Plattenteile zueinander ermöglicht. Dieser Teleskopmechanismus weist eine zwischen den beiden Plattenteilen eingespannte Zugfeder auf, die die beiden Plattenteile und damit die zu verbindenden Knochenfragmente aufeinander zu zieht, so daß der zwischen den Knochenfragmenten vorhandene Bruchspalt geschlossen wird. Auf diese Weise kann eine ansonsten eventuell auftretende interfragmentäre Diastase, d.h. ein Bruchspalt, verhindert werden, der zu einer Pseudarthrose führen kann.

Die Nachbehandlung von Tibiafrakturen ist unter anderem abhängig vom Schweregrad der Verletzung und von der gewählten Osteosyntheseform. Wird z.B. bei einer Tibiafraktur mit Biegungskeil in Schaftmitte eine Platte mit Schrauben gewählt und eine übungsstabile biologische Plattenosteosynthese erreicht, so kann eine Nachbehandlung unter physiotherapeutischer Anleitung nach etwa sechs bis acht Wochen erfolgen (Sohlenkontakt bei Entlastung an Stöcken). Durch Röntgenbilder kann zwar der Frakturdurchbau respektive die Frakturzonenfestigkeit annähernd erfaßt werden, wodurch die Festlegung des Belastungsbeginns (etwa 10 Kilogramm Teilbelastung ab der sechsten bis achten Woche) abgeschätzt werden kann, wobei jedoch die Beurteilung der Frakturzonenfestigkeit mittels Röntgenbild selbst für den Spezialisten schwierig und ungenau ist. Genauere Aussagen über die Frakturzonenfestigkeit können im Fall der Verwendung eines Fixateurs Externe zur Osteosynthese dann gemacht werden, wenn über die Verwindungssteifigkeit des Fixateurs auf die Frakturzonenfestigkeit rückgeschlossen wird.

Bis zum Belastungsbeginn sechs bis acht Wochen nach einer Operation treten Mikrobewegungen im Frakturbereich bei Verwendung einer Platte, und zwar auch einer Teleskopplatte mit Zugfeder gemäß der erwähnten CH-A-0 335 797, nur unkontrolliert und zufällig auf, z.B. bei der physio-therapeutischen Übungsbehandlung oder bei einer unbewußten oder ungewollten, die Extremität belastenden Bewegung des Patienten. Mikrobewegungen bestimmter Amplituden und/oder bestimmter Kraft können bei der biologischen Plattenosteosynthese bisher nicht kontrolliert in das Läsionsareal eingeleitet werden.

Aufgabe der vorliegenden Erfindung ist es, eine Platte zur Osteosynthese zu schaffen, mit der es ermöglicht wird, kontrollierte Mikrobewegungen in das Läsionsareal frakturierter Knochen in annähernd axialer und physiologischer Belastungsrichtung einzuleiten, um so die Kallogenese und damit den Heilungsprozeß zu fördern.

Diese Aufgabe ist gemaß der Erfindung durch die Merkmale des Patentanspruches 1 gelöst.

Der Teleskopmechanismus der Platte weist somit ein elastisches Bauteil auf, das derart angeordnet ist, daß es bei Annäherung der beiden Plattenteile zueinander komprimiert wird, dabei jedoch der Annäherung der beiden Plattenteile elastisch entgegenwirkt.

Bei einem bevorzugten Ausführungsbeispiel ist der Teleskopmechanismus als Flachteleskop ausgebildet und besteht aus je einer Verlängerung der beiden Plattenteile, wobei die eine Verlängerung als Hohlteil mit polygonalem Querschnitt ausgebildet ist und die andere Verlängerung als darin eingreifender Zapfen mit angepaßtem Querschnitt ausgebildet ist, so daß eine Relativverdrehung der beiden Plattenteile zueinander verhindert wird.

Bei einem anderen bevorzugten Ausführungsbeispiel ist der Teleskopmechanismus als Rundteleskop ausgestaltet, wobei jeweils zwei nebeneinander angeordnete Verlängerungen der beiden Plattenteile vorhanden sind und zwar zwei Hohlteile mit rundem Querschnitt des einen Plattenteils und zwei darin eingreifende (bzw. diese übergreifende) Zapfen mit rundem Querschnitt des anderen Plattenteils, so daß auch hier eine Relativverdrehung der beiden Plattenteile zueinander verhindert wird.

Besonders vorteilhaft ist es, wenn der Teleskopmechanismus eine Vorrichtung zur Begrenzung des Verschiebeweges der beiden Plattenteile aufweist, wobei diese Vorrichtung ebenfalls eine Vorabeinstellung des Verschiebeweges ermöglicht sowie eine Anordnung zur Einstellung der Druckvorspannung des elastischen Bauteils aufweist; die Vorrichtung kann dabei einen Abstandshalter aufweisen, der mit derjenigen Verlängerung des einen Plattenteils über eine Schraube fest verbunden ist, die in die Verlängerung des anderen Plattenteils eingreift; die aufnehmende Verlängerung des einen Plattenteils kann dabei mit einer Nut versehen sein, deren Länge in Verbindung mit der Länge des Abstandshalters den maximalen Verschiebeweg der beiden Plattenteile in Richtung zueinander bzw. voneinander weg begrenzt.

Weitere vorteilhafte Ausgestaltungen einer erfindungsgemäßen Platte gehen aus der nachfolgenden Beschreibung eines vorteilhaften Ausführungsbeispiels hervor. Es zeigen:

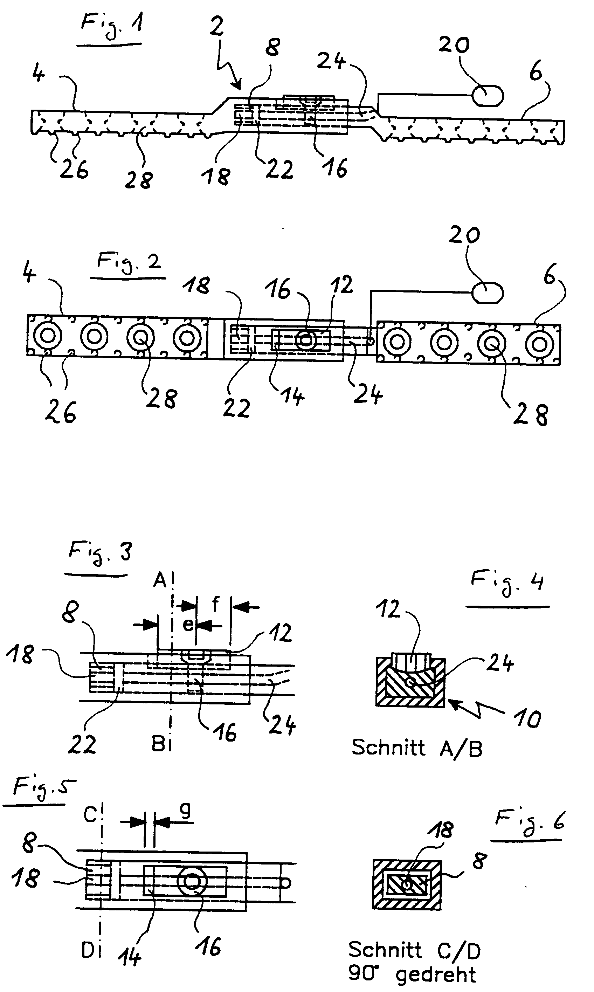

- Fig. 1: eine Seitenansicht einer erfindungsgemäßen Platte;
- Fig. 2: eine Draufsicht auf die erfindungsgemäße Platte;
- Fig. 3: eine vergrößerte Seitenansicht des Teleskopmechanismus;
- Fig. 4: einen Schnitt durch diesen Mechanismus entlang der Linie A/B;
- Fig. 5: eine Draufsicht auf den vergrößerten Teleskopmechanismus und
- Fig. 6: einen Schnitt entlang der Linie C/D von Fig. 5.

Die erfindungsgemäße Platte in Form einer Teleskopbrückenplatte dient zum Fixieren frakturierter Knochen und besteht aus zwei Plattenteilen 4, 6, die über einen Teleskopmechanismus 2, der sich vorzugsweise im Bereich der Frakturzone befindet, miteinander verbunden sind. Im Teleskopmechanismus ist ein Elastikelement 8 angeordnet, welches einer Annäherung der Plattenteile 4, 6 zueinander elastisch entgegenwirkt.

Bei einem ersten bevorzugten Ausführungsbeispiel besteht der Teleskopmechanismus 2 aus jeweils einem Ende der beiden Plattenteile 4 und 6 und ist als Flachteleskop 10 ausgebildet, wobei die eine Verlängerung eines der beiden Plattenteile als Hohlteil mit polygonalem Querschnitt ausgebildet sein kann, in das die Verlängerung des anderen Plattenteils, z.B. in Form eines eingreifenden Zapfens mit angepaßtem Querschnitt eingreift, so daß eine Relativverdrehung der beiden Plattenteile zueinander um ihre Verbindungsachse verhindert wird.

Bei einem anderen vorteilhaften Ausführungsbeispiel kann der Teleskopmechanismus als Rundteleskop ausgebildet sein, wobei je zwei nebeneinander angeordnete (nicht zeichnerisch dargestellte) Verlängerungen der beiden Plattenteile vorgesehen sind und zwar je zwei Hohlteile mit rundem Querschnitt des einen Plattenteils und zwei darin eingreifende Zapfen mit rundem Querschnitt des anderen Plattenteils, so daß auch hier eine Relativverdrehung der beiden Plattenteile zueinander um ihre Verbindungsachse verhindert wird.

Der Teleskopmechanismus 2 weist vorzugsweise eine Vorrichtung auf, um zum einen die Maximalbewegung der Plattenteile 4, 6 sowohl zueinander hin als auch voneinander weg vorab festzulegen, zum anderen die Maximalstrecke einer Bewegung der Plattenteile relativ zueinander vorab einzustellen und schließlich die Druckvorspannung des Elastikelementes einzustellen.

Im dargestellten Ausführungsbeispiel besteht diese Vorrichtung aus einem Abstandshalter 12, der mit der Verlängerung des einen Plattenteils 6 über eine Schraube 16 fest verbunden ist. Diese Verlängerung ist als inneres Flachteleskopteil ausgebildet. Die Verlängerung des anderen Plattenteils 4, welche als äußeres Teil des Flachteleskops 10 ausgebildet ist, bestimmt in Verbindung mit der Länge des Abstandshalters 12 die mögliche Maximalbewegung g der Plattenteile zueinander. Die Anordnung der Bohrung in der Verlängerung des Plattenteils 6 zur Aufnahme der Schraube 16 im Abstandshalter 12 bestimmt zwei Teilstrecken e und f. Dabei wird durch die Teilstrecke f das maximale Auseinanderweichen der Plattenteile 4 und 6 festgelegt und bei bestimmter Länge des Elastikelementes 8 eine bestimmte Vorspannung dieses Elastikelementes erzielt. Die Teilstrecken e und f bestimmen auch die Länge des Abstandshalters 12 und legen somit eine mögliche Maximalbewegung g der Plattenteile 4, 6 zueinander fest. Durch Variation der Länge des Abstandshalters 12 und durch Variation der Lokalisation der Bohrung im Abstandshalter kann sowohl die Maximalbewegung der Plattenteile 4 und 6 zueinander hin und voneinander weg festgelegt werden, als auch die Maximalstrecke einer Bewegung der Plattenteile relativ zueinander eingestellt werden und schließlich die Druckvorspannung des Elastikelementes 8 festgelegt werden.

Das Elastikelement 8 kann aus einem Kunststoff oder aber einer Feder oder aber einer Kombination aus diesen beiden bestehen. Vorzugsweise weist das Elastikelement 8 verschiedene Materialien unterschiedlicher Elastizitätsmodule auf, so daß bei einer bestimmten Längenänderung des Elastikelementes 8 eine vorgegebene Widerstandskraft gegen Verformung auftritt.

Bei dem in den Figuren dargestellten Ausführungsbeispiel ist ferner mit 18 ein Wegaufnehmer bezeichnet, der mit einem Sender 20 zusammenwirkt, um kontinuierlich oder auch in kleinsten Zeitabständen z.B. Mikrosekunden oder Millisekunden Wegänderungen bzw. Relativbewegungen der Plattenteile 4 und 6 sowohl in Richtung zueinander hin als auch voneinander weg über Telemetrie oder Infrarottelemetrie an einen extrakorporalen Empfänger zu senden. Der Sender 20 kann passiv z.B. durch Stimulation von außen oder aktiv mit implantierter Energiequelle ausgebildet sein.

Ferner ist mit 22 ein Druckaufnehmer bezeichnet, der z.B. zwischen dem Elastikelement 8 und einem der Plattenteile 6 angebracht werden kann, um möglichst exakt die Kraft zu bestimmen, die bei einer Axialbewegung der Plattenteile relativ zueinander letztendlich auftritt. Auch der Druckaufnehmer 22 sollte an den passiven oder aktiven Sender 20 angekoppelt werden, um seine Meßwerte z.B. mittels Telemetrie an einen Empfänger senden zu können. So ist es ohne weiteres mittels der bekannten Multiplextechnik möglich auch in kleinsten Zeitabständen die Signale sowohl vom Wegaufnehmer als auch vom Druckaufnehmer über einen gemeinsamen Sender einem gemeinsamen Empfänger zuzuführen.

Mit 24 ist eine Längsbohrung in der Verlängerung des Plattenteils 6 bezeichnet, durch die z.B. eine Kabelverbindung zwischen Druckaufnehmer 22 und Wegaufnehmer 18 zum Sender 20 hergestellt werden kann.

Um ferner eine mögliche Verbiegung oder eine auftretende Verdrehung der Teleskopbrückenplatte unter Belastung messen zu können, ist mindestens ein nicht dargestellter Dehnungsmeßstreifen, z.B. ein kleiner Halbleiterdehnungsmeßstreifen im Bereich der Bohrung 24 angeordnet. Sollte der Teleskopmechanismus 2 bei Belastung durch Verkanten klemmen, könnte durch eine Messung der dabei auftretenden Verdrehung oder Verkantung zwischen den beiden Plattenteilen 4, 6 eine akustische und/oder optische Warnanlage betätigt werden. Ferner könnte eine Beanspruchung der Platte bei Belastung auch zur Feststellung der Frakturzonenfestigkeit verwendet werden. Wird nur eine geringe oder gar keine Verbiegung oder Verdrehung der Platte bei Belastung festgestellt, so kann dies auf eine gefestigte Frakturzone hinweisen, oder aber eine gelockerte oder gebrochene Platte signalisieren.

Auch der Dehnungsmeßstreifen sollte an einen passiven oder aktiven Sender, z.B. den Sender 20, gekoppelt werden, um seine Meßwerte mittels Telemetrie einem Empfänger zuzuführen; auch hierfür eignet sich die bekannte Multiplextechnik.

Die erfindungsgemäße Platte zur Osteosynthese mit dem Teleskopmechanismus und den zugehörigen Sensoren und Sendern eignet sich insbesondere dazu, im Zusammenhang mit einer an der Extremität angebrachten Stimulationsplatte verwendet zu werden, welche Signale von einer Regeleinheit erhält, der wiederum die Ausgangssignale der Druckaufnehmer, Wegaufnehmer, Dehnungsmesser zugeführt werden. Die Stimulationsplatte kann dann in Abhängigkeit der ihr zugeführten Signale Mikrobewegungen in den Läsionsbereich einleiten, wobei die Bewegungen der Extremität durch den erfindungsgemäßen Teleskopmechanismus ermöglicht werden. Ein Zusammendrücken des im Teleskopmechanismus 2 vorhandenen Elastikelementes 8 sorgt bei nachlassender Krafteinleitung dafür, daß die Plattenteile wieder in ihre Ausgangsstellung zurückkehren.

Wird durch eine externe Kraft, z.B. durch eine (nicht dargestellte) Stimulationsplatte eine Axialbewegung der Plattenteile zueinander hin oder voneinander weg hervorgerufen, so kann über die Weg- und Kraftmessungen an der Platte bei bekanntem Elastizitätsmodul des Elastikelementes 8 auf die Frakturzonenfestigkeit geschlossen werden und so das Heilungsstadium angezeigt werden. Bei fortschreitender Festigkeitsentwicklung der Frakturzone kann demzufolge eine zunehmende Belastung der Extremität unter gleichzeitiger Berücksichtigung der Stimulationsamplitude erfolgen. Bei kleiner werdender Amplitude sollte eine größer werdende Stimulationskraft verwendet werden.

Die erfindungsgemäße Platte kann aus biokompatiblem Material hoher Biegesteifigkeit, z.B. aus Titan oder Titanlegierungen bestehen. Die dem Knochen zugewandte Auflageseite der beiden Plattenteile besteht dabei vorteilhafterweise aus mehreren, in diesem Ausführungsbeispiel pro Plattenteil achtzehn, hervorspringenden kleinen Pfeilern 26, damit die Vitalität des Knochens nicht durch große Auflageflächen beeinflußt wird. Der Teleskopmechanismus 2 sollte dabei nicht auf dem Knochen aufliegen, um seine freie Bewegung nicht zu zerstören. In den Plattenteilen selbst sind ferner mindestens zwei Öffnungen 28 vorhanden, um die Plattenteile mittels versenkbarer und schwenkbarer Schrauben in den Knochenfragmenten zu befestigen.

Anstelle einer Stimulationsplatte zur Einleitung der Mikrobewegungen kann auch ein Muskelstimulationsgerät eingesetzt werden.

Vom Patienten selbst ausgeführte Belastungen oder Muskelkontraktionen können ebenfalls über die erfindungsgemäße Platte erfaßt, an einen Rechner weitergeleitet und auf einem Bildschirm dargestellt werden. Etwa eine Woche nach Beginn der Osteosynthese sollte mit Hilfe der erfindungsgemäßen Platte eine mechanische Stimulation im Läsionsareal beginnen, wonach bei Einsatz z.B. einer Stimulationsplatte in einem Regelkreis zusammen mit einem Rechner für das sich zum Knochen differenzierende Reparationsgewebe zu jedem Zeitpunkt der Frakturheilung eine optimale biomechanische Umgebung geschaffen werden kann. Eine Verkürzung der Heilungszeit um mehrere Wochen ist mit dieser Methode zu erzielen.

## Patentansprüche

1. Platte zur Osteosynthese frakturierter Röhrenknochen, die mit einer Vielzahl von Schrauben versehen ist zur Verankerung in den miteinander zu verbindenden Fragmenten, wobei die Platte aus zwei in Längsrichtung hintereinander angeordneten Plattenteilen (4, 6) besteht, die über einen Teleskopmechanismus (2) miteinander verbunden sind, welcher axiale Relativbewegungen der beiden Platten zueinander ermöglicht und zwischen den Plattenteilen (4, 6) ein elastisches Bauteil (8) aufweist, dadurch gekennzeichnet, daß zur Einleitung kontrollierter Bewegungen der beiden Plattenteile (4, 6) und damit der Knochenfragmente zueinander in physiologischer Belastungsrichtung das elastische Bauteil (8) so ausgebildet ist, daß es einer Annäherung der beiden Plattenteile (4, 6) elastisch entgegenwirkt.

2. Platte nach Anspruch 1, dadurch gekennzeichnet, daß der Teleskopmechanismus (2) ein Flachteleskop ist, das je eine Verlängerung der beiden Plattenteile aufweist, und zwar ein Hohlteil mit polygonalem Querschnitt des einen Plattenteils und einen darin eingreifenden Zapfen mit angepaßtem Querschnitt des anderen Plattenteils, so daß eine Relativverdrehung der beiden Plattenteile zueinander verhindert wird.

3. Platte nach Anspruch 1, dadurch gekennzeichnet, daß der Teleskopmechanismus ein Rundteleskop ist, das je zwei nebeneinander angeordnete Verlängerungen der beiden Plattenteile aufweist, und zwar zwei Hohlteile mit rundem Querschnitt des einen Plattenteils und zwei darin eingreifende Zapfen mit rundem Querschnitt des anderen Plattenteils, so daß eine Relativverdrehung der beiden Platten zueinander verhindert wird.

4. Platte nach einem der vorhergehenden Ansprüche, dadurch gekennzeichnet, daß der Teleskopmechanismus eine Vorrichtung zur Begrenzung des Verschiebeweges der beiden Plattenteile aufweist.

5. Platte nach Anspruch 4, dadurch gekennzeichnet, daß die Vorrichtung eine Anordnung zur Vorabeinstellung des Verschiebeweges aufweist.

6. Platte nach Anspruch 4, dadurch gekennzeichnet, daß die Vorrichtung eine Anordnung zur Einstellung der Druckvorspannung des elastischen Bauteils aufweist.

7. Platte nach Ansprüchen 4 bis 6, dadurch gekennzeichnet, daß die Vorrichtung einen Abstandshalter (12) aufweist, der mit derjenigen Verlängerung des einen Plattenteils (6) über eine Schraube (16) fest verbunden ist, die in die Verlängerung des anderen Plattenteils (4) eingreift.

8. Platte nach Anspruch 7, dadurch gekennzeichnet, daß die die Verlängerung des Plattenteils (6) aufnehmende Verlängerung des Plattenteils (4) mit einer Nut (14) versehen ist, deren Länge in Verbindung mit der Länge des Abstandshalters (12) den maximalen Verschiebeweg (g) der Plattenteile (4, 6) relativ zueinander begrenzt.

9. Platte nach Anspruch 1, dadurch gekennzeichnet, daß das Elastikelement (8) aus verschiedenen Materialien unterschiedlicher Elastizitätsmodule zusammengesetzt ist.

10. Platte nach einem der vorhergehenden Ansprüche, dadurch gekennzeichnet, daß der Teleskopmechanismus mit einem Wegaufnehmer (18) versehen ist, der mit einem Sender (20) verbunden ist.

11. Platte nach einem der vorhergehenden Ansprüche, dadurch gekennzeichnet, daß der Teleskopmechanismus mit einem Druckaufnehmer (22) versehen ist, der zwischen dem Elastikelement (8) und einem der beiden Plattenteile (6) angeordnet ist und der mit einem Sender (20) verbunden ist.

12. Platte nach einem der vorhergehenden Ansprüche, dadurch gekennzeichnet, daß der Teleskopmechanismus mit einem Dehnungsmeßstreifen versehen ist, der über einen Sender mit einer Alarmanlage verbunden ist, um Relativverdrehungen der Plattenteile zueinander um die Teleskopachse anzuzeigen oder um ein Verbiegen oder Verdrehen der Plattenteile anzuzeigen.

13. Platte nach den Ansprüchen 10 und 11, dadurch gekennzeichnet, daß der Teleskopmechanismus mit einer Regeleinheit verbunden ist, die in Abhängigkeit von den Meßwerten des Druckaufnehmers und des Wegaufnehmers Steuersignale an eine Stimulationsplatte oder ein Muskelstimulationsgerät überträgt, welche mit der zu behandelnden Extremität verbunden sind und durch Krafteinleitung in die Extremität genau dosierte Mikrobewegungen im Frakturbereich zur Induktion der Osteosynthese erzeugt.

14. Platte nach einem der vorhergehenden Ansprüche, dadurch gekennzeichnet, daß die Plattenteile aus Titan oder einer Titanlegierung bestehen und daß die dem Knochen zugewandte Auflageseiten der Plattenteile mehrere hervorspringende Pfeiler (26) aufweisen.

## Claims

1. Plate for the osteosynthesis of fractured long bones, which is provided with a plurality of screws for anchoring in the fragments to be joined together, wherein the plate consists of two plate parts (4, 6) disposed one behind another in the longitudinal direction which are connected together by a telescope mechanism (2), which permits axial relative motions of the two plates and has between the plate parts (4, 6) a resilient component (8), characterised in that in order to produce controlled movements of the two plate parts (4, 6) and thereby of the bone fragments relative to one another in the physiological load direction the resilient component (8) is so formed that it resiliently counteracts an approach of the two plate parts (4, 6) relative to one another.

2. Plate according to claim 1, characterised in that the telescope mechanism (2) is a flat telescope, which has a respective extension for each of the two plate parts, in particular a hollow part of polygonal cross-section of the one plate part and a pin engaging therein with a suitable cross-section of the other plate part, so that relative rotation of the two plate parts is prevented.

3. Plate according to claim 1, characterised in that the telescope mechanism is a round telescope, which has two extensions for each of the two plate parts disposed one next to the other, in particular two hollow parts with a round cross-section of the one plate part and two pins engaging therein of round cross-section of the other plate part, so that relative rotation of the two plate parts is prevented.

4. Plate according to one of the preceding claims, characterised in that the telescope mechanism has a device for limiting the displacement distance of the two plate parts.

5. Plate according to claim 4, characterised in that the device has an arrangement for presetting the displacement distance.

6. Plate according to claim 4, characterised in that the device has an arrangement for setting the pressure prestressing of the resilient component.

7. Plate according to claims 4 to 6, characterised in that the device has a brace (12) which is rigidly connected to the extension of the plate part (6) via a screw (16), which engages in the extension of the other plate part (4).

8. Plate according to claim 7, characterised in that the extension of the plate part (4) receiving the extension of the plate part (6) is provided with a groove (14), whose length in combination with the length of the brace (12) defines the maximum displacement distance (g) of the plate parts (4, 6) relative to one another.

9. Plate according to claim 1, characterised in that the resilient element (8) is composed of various materials of different moduli of elasticity.

10. Plate according to one of the preceding claims, characterised in that the telescope mechanism is provided with a displacement transducer (18) which is connected to a transmitter (20).

11. Plate according to one of the preceding claims, characterised in that the telescope mechanism is provided with a pressure sensor (22) which is disposed between the resilient element (8) and one of the two plate parts (6) and which is connected to a transmitter (20).

12. Plate according to one of the preceding claims, characterised in that the telescope mechanism is provided with an expansion measuring strip, which is connected via a transmitter to an alarm device in order to indicate relative rotation of the plate parts about the telescope axis or to indicate bending or twisting of the plate parts.

13. Plate according to claims 10 and 11, characterised in that the telescope mechanism is connected to a control unit, which as a function of the measurements of the pressure sensor and of the displacement transducer transmits control signals to a stimulation plate or muscle stimulation device, which are connected to the end to be treated, and generates by power induction in the end precisely metered micro-movements in the fracture region in order to induce osteosynthesis.

14. Plate according to one of the preceding claims, characterised in that the plate parts consist of titanium or a titanium alloy and in that the contact sides of the plate parts facing the bone have a plurality of projecting columns (26).

## Revendications

1. Plaque pour l'ostéosynthèse des os fracturés de forme tubulaire, pourvue d'une pluralité de vis pour l'ancrage des fragments à relier entre eux, la plaque étant constituée de deux portions de plaque (4, 6) accouplées dans le sens longitudinal et qui sont reliées entre elles par un mécanisme télescopique (2), qui permet un mouvement axial relatif des deux plaques l'une par rapport à l'autre et qui présente un élément élastique (8) entre les deux portions de plaque (4, 6), caractérisée en ce que, pour l'introduction, dans la direction de sollicitation physiologique, de mouvements contrôlés des deux portions de plaque (4, 6) et par conséquent des fragments d'os, l'élément élastique (8) est conformé de façon qu'il s'oppose élastiquement à un rapprochement des deux portions de plaque (4, 6).

2. Plaque selon la revendication 1, caractérisée en ce que le mécanisme télescopique (2) est un télescope plat, qui consiste en des prolongements de chacune des portions de plaque, à savoir en une partie creuse de section polygonale de l'une des portions de plaque dans laquelle vient s'engager un tenon de section adaptée de l'autre portion de plaque, de façon à empêcher une torsion relative des deux portions de plaque l'une par rapport à l'autre.

3. Plaque selon la revendication 1, caractérisée en ce que le mécanisme télescopique est un télescope à section ronde, qui consiste en deux prolongements de chacune des portions de plaque disposés de front, à savoir en deux parties creuses de sections rondes de l'une des portions de plaque dans lesquelles viennent s'engager deux tenons de sections rondes de l'autre portion de plaque, de façon à empêcher une torsion relative des deux plaques l'une par rapport à l'autre.

4. Plaque selon l'une des revendications précédentes, caractérisée en ce que le mécanisme télescopique constitue un dispositif pour délimiter le chemin de déplacement des deux portions de plaque.

5. Plaque selon la revendication 4, caractérisée en ce que le dispositif présente une installation pour le préréglage de la course de déplacement.

6. Plaque selon la revendication 4, caractérisée en ce que le dispositif présente une installation pour le réglage de la prétension d'appui de l'élément élastique.

7. Plaque selon l'une des revendications 4 à 6, caractérisée en ce que le dispositif présente une entretoise (12), qui est solidement fixée audit prolongement de l'une des portions de plaque (6) au moyen d'une vis (16) et qui engrène sur le prolongement de l'autre portion de plaque (4).

8. Plaque selon la revendication 7, caractérisée en ce que le prolongement de la portion de plaque (6) qui accueille le prolongement de la portion de plaque (4) est pourvu d'une rainure (14), dont la longueur en relation avec la longueur de l'entretoise (12) limite le chemin de déplacement relatif maximum (g) des portions de plaque (4, 6) l'une par rapport à l'autre.

9. Plaque selon la revendication 1, caractérisée en ce que l'élément élastique (8) est composé de différents matériaux présentant des modules d'élasticité différenciés.

10. Plaque selon l'une des revendications précédentes, caractérisée en ce que le mécanisme télescopique est pourvu d'un capteur de déplacement (18) relié à un émetteur (20).

11. Plaque selon l'une des revendications précédentes, caractérisée en ce que le mécanisme télescopique est pourvu d'un capteur de pression (22) disposé entre l'élément élastique (8) et l'une des portions de plaque (6) et relié à un émetteur (20).

12. Plaque selon l'une des revendications précédentes, caractérisée en ce que l'élément télescopique est pourvu d'une jauge extensométrique reliée à un dispositif d'alarme au moyen d'un émetteur, de façon à signaler des torsions relatives des portions de plaque l'une par rapport à l'autre autour de l'axe du télescope ou pour signaler une déformation ou une torsion des portions de plaque.

13. Plaque selon les revendications 10 et 11, caractérisée en ce que le mécanisme télescopique est relié à une unité de réglage, qui, en fonction des valeurs mesurées par le capteur de pression et le capteur de déplacement, transmet des signaux de commande à une plaque de stimulation ou à un appareil de stimulation musculaire, qui sont reliés avec l'extrémité à traiter et engendrent des micro-mouvements dosés exactement par l'introduction de forces dans les extrémités dans la région de la fracture pour l'induction de l'ostéosynthèse.

14. Plaque selon l'une des revendications précédentes, caractérisée en ce que les portions de plaque sont constituées en titane ou en alliage de titane et en ce que les parties d'appui des portions de plaque jouxtant l'os présentent plusieurs jambages (26) faisant saillie.
